# FASCICULE DE BREVET EUROPEEN

(11) **EP 1 127 072 B1**
(45) Date de publication et mention de la délivrance du brevet: **30.04.2008**
(21) Numéro de dépôt: 00960796.1
(22) Date de dépôt: 05.09.2000
(51) Int. Cl.: C07K 14/705, G01N 33/68, C12N 5/08

(54) **MOYENS DE DETECTION ET DE PURIFICATION DE POPULATIONS LYMPHOCYTAIRES TCD8+, SPECIFIQUES DE PEPTIDES PRESENTES DANS LE CONTEXTE HLA**
MITTEL ZUR BESTIMMUNG UND REINIGUNG VON LYMPHOCYTENPOPULATIONEN DES TYPS TCD8+, DIE SPEZIFISCH FÜR IM ZUSAMMENHANG MIT HLA PRÄSENTIERTE PEPTIDE SIND
METHOD FOR DETECTING AND PURIFYING TCD8+ LYMPHOCYTE POPULATIONS, SPECIFIC OF PEPTIDES PRESENT IN THE CONTEXT OF HLA

(30) Priorité: 06.09.1999 FR 9911133
(43) Date de publication de la demande: 29.08.2001
(73) Titulaire: INSTITUT NATIONAL DE LA SANTE ET DE LA RECHERCHE MEDICALE (INSERM), 75654 Paris Cédex 13 (FR)
(72) Inventeur: LANG, François, F-44000 Nantes (FR); BODINIER, Marie, F-44000 Nantes (FR); DAVODEAU, François, F-44000 Nantes (FR); BONNEVILLE, Marc, F-44120 Vertou (FR)
(74) Mandataire: Peaucelle, Chantal
(86) Numéro de dépôt international: PCT/FR2000/002443
(87) Numéro de publication internationale: WO 2001/018053

(56) Documents cités:
- WO-A-96/26962
- WO-A-97/44667
- WO-A-99/11775
- R.D. SALTER ET AL.: "A binding site for the T-cell co-receptor CD8 on the alpha3 domain of HLA-A2" NATURE, vol. 345, 3 mai 1990 (1990-05-03), pages 41-46, XP002137488 LONDON GB cité dans la demande

### LISTE DE SEQUENCES

<110> I.N.S.E.R.M.

<120> MOYENS DE DETECTION ET DE PURIFICATION DE POPULATIONS LYMPHOCYTAIRES TCD8+, SPECIFIQUES DE PEPTIDES PRESENTES DANS LE CONTEXTE HLA.
<130> CP/VB/1195FR99
<140>
<141>
<160>2
<170> Patentin Ver. 2.1
<210> 1
<211>31
<212> ADN
<213> Séquence artificielle
<220>
<223> Description de la séquence artificielle:région
   homologue à une partie du gène HLA-A0201 humain.
<400> 1
   ccttccagaa gtgggtggct gtggtggtgc c 31
<210>2
<211>31
<212> ADN
<213> Séquence artificielle
<220>
<223> Description de la séquence artificielle:région
   homologue à une partie du gène HLA-A0201 humain.
<400> 2
   ggcaccacca cagccaccca cttctggaag g 31

## Description

L'invention a pour objet des moyens de détection et de purification de populations lymphocytaires TCD8+, spécifiques de peptides présentés dans le contexte HLA.

Le lymphocyte T porte un récepteur spécifique de l'antigène contre lequel il est commis, appelé TCR. Ce TCR est composé de plusieurs chaînes, dont les chaînes α et β qui sont impliquées dans la reconnaissance spécifique d'un peptide antigénique particulier présenté dans une molécule HLA. Cette reconnaissance se matérialise par la capacité du TCR α/β du lymphocyte T à se fixer avec une certaine affinité aux complexes HLA-peptide présents à la surface de la cellule cible. Réciproquement, des complexes HLA-peptide solubles sont capables de se fixer aux TCR présents à la surface des lymphocytes T spécifiques du complexe HLA-peptide considéré.

A l'heure actuelle, le système le mieux étudié moléculairement est la reconnaissance par des lymphocytes T CD8+ de peptides antigéniques présentés dans des molécules du complexe majeur d'histocompatibilité (CMH) de classe I et en particulier dans l'allèle HLA-A0201.

Dans ce système, il est établi que l'affinité du TCR pour le complexe HLA-peptide est très faible comparée à l'affinité d'un anticorps pour son antigène. Pour cette raison, la détection de lymphocytes porteurs de TCR réactifs contre un peptide spécifique dans ce contexte HLA à l'aide de molécules HLA-A0201 solubles chargées en peptides et marquées est impossible. Pour pallier cette faible affinité, Altman et al (1) ont fabriqué un réactif multivalent composé de complexes HLA-A0201-peptide où la chaîne lourde du HLA est biotinylée, ce qui permet une association en tétramère avec la streptavidine. Ce tétramère HLA-peptide présente une avidité augmentée pour les lymphocytes T porteurs des TCR appropriés et peut donc être utilisé pour visualiser par immunofluorescence les populations réactives.

Mais le TCR n'est pas la seule molécule du lymphocyte T qui puisse interagir avec le complexe HLA-peptide. En effet, lors de la reconnaissance physiologique, la fixation du TCR au complexe CMH-peptide est renforcée par la fixation du co-récepteur CD8 à une portion constante des molécules CMH de classe I. La participation du CD8 dans l'interaction est variable d'un clone lymphocytaire à l'autre et peut dans certains cas conduire à une augmentation très importante de la capacité de fixation à un complexe HLA-peptide donné. Cette capacité du CD8 à se fixer au HLA de classe I a pour conséquence d'entraîner un bruit de fond de fixation des tétramères HLA de classe I sur des lymphocytes T CD8+ qui portent des TCR non spécifiques du complexe HLA-peptide. Ce bruit de fond augmente avec la concentration de tétramère utilisée et peut conduire à des faux positifs en immunofluorescence. Pour essayer de diminuer ce marquage non spécifique, la plupart des équipes réalisent leurs marquages avec les tétramères HLA de classe I en présence d'anticorps anti-CD8. Mais seuls certains anticorps anti-CD8 sont efficaces et les rapports optimaux de concentrations entre l'anticorps et le tétramère doivent être réajustés à chaque test. Du fait de ces inconvénients, la détection de sous-populations spécifiques peu représentées au sein d'une population non spécifique (par exemple de l'ordre de 0,1 à 1 %) devient difficile.

Par ailleurs, une autre application potentielle des tétramères HLA a été envisagée. Elle consiste à isoler par tri (en cytométrie de flux ou par tri immunomagnétique) des populations lymphocytaires réactives contre un complexe HLA-peptide donné à des fins d'expansion *in vitro,* puis d'utilisation thérapeutique dans des protocoles d'immunisation passive anti-virale ou anti-tumorale. Mais le bruit de fond de fixation du tétramère dû à la participation du CD8 peut constituer un obstacle sérieux à cette application car il conduit à l'isolement d'une fraction souvent importante de lymphocytes T non réactifs vis-à-vis du complexe HLA-peptide sélectionnant.

Salter et al (2) ont montré que la fixation d'un HLA membrannaire exprimé par des cellules transfectées à un co-récepteur CD8αα était altérée lorsque le HLA comportait une mutation dans le domaine α3.

L'étude de telles mutations par les inventeurs les a conduits à vérifier que des tétramères mutés solubles fixent effectivement moins le CD8, qu'il soit αα ou αβ, associé ou non à un TCR à la surface du lymphocyte T, ce qui se traduit par une diminution du bruit de fond.

On pouvait alors s'attendre à ce que la perte d'affinité résultant de la mutation conduise à une perte de signal spécifique totale ou restreinte à certains clones lymphocytaires T CD8 dépendants. L'article de Salter et al montre à cet égard que certains clones alloréactifs CD8 dépendants perdent leur cytotoxicité vis-à-vis de cellules portant le HLA-A2 muté tandis que d'autres sont peu affectés.

Ainsi il aurait été possible que les tétramères mutés ne détectent qu'une fraction des cellules réactives (les moins CD8 dépendantes) au sein d'une population polyclonale.

Les nombreux marquages comparatifs de populations polyclonales avec les tétramères mutés et natifs réalisés par les inventeurs en double marquage avec un anticorps anti-CD8 démontrent qu'au contraire, de manière inattendue, le tétramère muté reconnaît le même pourcentage de cellules spécifiques que le tétramère natif.

De plus, la comparaison de marquage avec le tétramère muté sur un clone très CD8 dépendant et un clone peu CD8 dépendant montre une efficacité comparable de fixation du tétramère rapportée à l'intensité d'expression du TCR.

I1 apparaît donc que la mutation diminue de façon très importante la fixation du tétramère au CD8 seul, mais affecte beaucoup moins sa fixation au complexe TCR-CD8.

L'invention repose donc sur la mise à profit des propriétés mises en évidence chez les multimères de HLA mutés, ou, de manière plus générale, altérés, et vise, en tant que nouveaux produits, de tels multimères et leurs complexes avec des peptides antigéniques.

Elle vise également l'utilisation de ces molécules pour la détection et/ou l'isolement de populations lymphocytaires TCD8+, peptide-spécifiques.

Elle vise en outre à fournir une méthode de détection et/ou d'isolement de telles populations à l'aide de telles molécules, chargées en peptide, en particulier pour des applications en diagnostic et en thérapeutique.

L'invention vise ainsi un complexe tétramérique de monomères de CMH de classe I ayant au moins une substitution d'un acide aminé dans le domaine α3 dans la zone d'interaction d'une chaîne lourde de monomère de CMH de classe I avec un co-récepteur CD8 des lymphocytes T, l'affinité de ce complexe tétramérique pour les co-récepteurs CD8 des lymphocytes T étant réduite par rapport à l'affinité d'un complexe tétramérique natif non substitué correspondant.

L'altération de la zone d'interaction concerne plus spécialement le domaine α3 de la chaîne lourde.

Il s'agit tout particulièrement d'une mutation dans le domaine α3 d'au moins un acide aminé, par rapport au domaine correspondant d'une chaîne lourde native capable de se fixer audit co-récepteur CD8.

On citera par exemple la mutation d'un résidu alanine en résidu valine en position 245 du domaine α3 de la molécule HLA-A2.

L'altération peut également consister en une modification chimique d'au moins un acide aminé et/ou en une délétion d'au moins un acide aminé, ce ou ces types d'altération s'ajoutant le cas échéant à une ou plusieurs mutations.

L'invention vise également, en tant que nouveaux produits, les complexes élaborés à partir des multimères définis ci-dessus et de peptides antigéniques.

Dans ces complexes, les multimères se présentent notamment sous forme de tétramères.

Conformément à l'invention, ces complexes sont utilisés pour la détection et/ou l'isolement de populations lymphocytaires TCD8+ reconnaissant de manière spécifique le peptide antigénique des complexes.

L'utilisation des complexes définis ci-dessus permet de diminuer de façon très importante le bruit de fond de fixation non spécifique sans altérer le marquage spécifique et, en outre, de s'affranchir de la nécessité d'utiliser conjointement un anticorps anti-CD8 lors d'analyses en immunofluorescence.

Dans une utilisation préférée, lesdits complexes sont mis en oeuvre dans un procédé de tri cellulaire, tel que le tri immunomagnétique.

Une technique de tri immunomagnétique pour isoler des lymphocytes T spécifiques chez la souris est décrite par Luxembourg et al. (5).

Cette technique est basée sur l'utilisation d'un système de billes recouvertes de complexes CMH-peptide (produits dans un système Drosophile ; chargés en peptides et biotinylés chimiquement).

L'invention vise également une méthode de détection et/ou d'isolement de populations lymphocytaires T CD8+ peptide-spécifiques, à partir d'une population polyclonale. La méthode de détection est caractérisée en ce qu'elle comprend :
- la mise en contact de la population polyclonale avec des multimères complexés à des peptides antigéniques tels que définis ci-dessus, dans des conditions permettant une interaction entre les complexes de CMH de classe I altérés/peptides et les récepteurs des lymphocytes T ayant une affinité pour lesdits complexes,
- la révélation des populations lymphocytaires qui se sont fixées auxdits complexes.

La révélation est réalisée par exemple par fluorescence en utilisant des multimères comportant des composés fluorescents.

La méthode d'isolement des populations lymphocytaires peptide-spécifiques à partir de populations polyclonales entre également dans le cadre de l'invention et peut être mise en oeuvre le cas échéant après l'étape de détection ci-dessus.

Cette méthode met en jeu la technique du tri immunomagnétique et est caractérisée en ce que qu'elle comprend :
- la mise en contact de la population polyclonale avec des billes magnétiques sur lesquelles sont fixées les complexes peptide/analogues de CMH de classe I, tels que définis ci-dessus, dans des conditions permettant une interaction entre lesdits complexes et les récepteurs des lymphocytes T ayant une affinité pour ces complexes,
- la récupération des populations fixées, l'opération de tri étant répétée, si souhaité, et/ou suivie, le cas échéant, d'une étape
- d'amplification *in vitro* des populations sélectionnées.
   L'augmentation du différenciel entre bruit de fond et marquage spécifique avec les multimères altérés comme défini ci-dessus permet une meilleure discrimination des populations lymphocytaires spécifiques au sein d'une population polyclonale, et rend ainsi très efficace le tri immunomagnétique avec ces multimères.
   Les molécules de CMH comprennent par exemple un motif de biotinylation enzymatique sur la chaîne lourde. Les billes sur lesquelles sont fixées les complexes sont couplées à la streptavidine.
   Les populations polyclonales proviennent d'échantillons prélevés sur des patients, comme des liquides synoviaux ou des cellules mononuclées du sang périphérique.
   L'étape d'amplification des populations sélectionnées est réalisée avantageusement par stimulation polyclonale dans des conditions qui n'affectent pas la représentativité des populations amplifiées. On a recours par exemple à PHA, IL2 ou des PBL irradiés.
   L'invention vise également les populations lymphocytaires T sélectionnées et le cas échéant amplifiées, caractérisées en ce qu'elles sont exclusivement constituées de lymphocytes T réactifs vis-à-vis du peptide d'un complexe donné.
   De telles populations peptide-spécifiques revêtent un grand intérêt en thérapeutique, et plus spécialement pour les applications qui reposent sur leur ré-admnistration selon l'immunothérapie adoptive.
   L'invention vise donc des compositions pharmaceutiques caractérisées en ce qu'elles sont élaborées à partir d'une population lymphocytaire T peptide-spécifique telle que définie ci-dessus, en association avec un véhicule pharmaceutique.
   De telles compositions sont avantageusement administrables par injection.
   Il est ainsi possible de restaurer une immunité antivirale ou antitumorale après injection de lymphocytes T reconnaissant respectivement un complexe CMH de classe I/peptide viral ou tumoral défini, ou de corriger un désiquilibre immunitaire (par exemple cas d'autoimmunité,) par l'administration de cellules T dirigées contre un antigène donné et présentant des propriétés activatrices ou inhibitrices de la réponse immunitaire.
   D'autres caractéristiques et avantages de l'invention figurent dans les exemples qui suivent, donnés à titre purement illustratif, dans lesquels il est fait référence aux figures 1 à 7, qui représentent, respectivement
- les figures 1 et 2 représentent les intensités de fluorescence (moyennes) en fonction des concentrations en tétramères natifs (1A, 2A) et en tétramères mutés correspondants (1B, 2B) avec des tétramères chargés en peptide issu de BMLF1 (fig.1) ou de pp65 (fig. 2),
- la figure 3, les moyennes d'intensité de fluorescence en fonction des concentrations en tétramères natifs et mutés avec des clones spécifiques,
- la figure 4A, le pourcentage de lyse, avec ou sans anticorps anti-CD8 de 2 clones spécifiques d'une lignée B HLA-AO201 chargée avec un peptide BMLF1 et les figures 4A et 4B, les résultats des marquages de ces clones,
- la figure 5, les résultats de simples (avec le phycoérythrine, PE) et doubles (PE et anticorps anti-CD8 marqués) marquages de tétramères natifs et mutés avec les lymphocytes de 2 patients, et
- les figures 6 et 7, les résultats de tris immunomagnétiques réalisés avec des tétramères natifs et des tétramères mutés.

### Exemple 1 : Fabrication d'un tétramère HLA-A0201 muté dans la zone d'interaction avec le co-récepteur CD8.

On a utilisé un plasmide d'expression en bactérie contenant l'ADNc codant pour la chaîne lourde du HLA0201, prolongé d'une séquence codant pour un motif de biotinylation enzymatique (construction selon Altman et al (1). La zone codant pour le domaine α3 a été amplifiée avec des amorces spécifiques SEQ ID N°1

5' CCTTCCAGAAGTGGGTGGCTGTGGTGGTGCC 3'

et SEQ ID N°2

5' GGCACCACCACAGCCACCCACTTCTGGAAGG 3'

On a introduit dans les fragments d'amplification une mutation d'une base pour transformer le codon alanine en un codon valine en utilisant le kit QuickChange Site-directed Mutagenesis R de Stratagène.

Le fragment muté a été réintroduit dans le plasmide d'expression et la présence de la mutation a été contrôlée par séquençage.

La chaîne lourde HLA-A0201 mutée a été produite en corps d'inclusion bactérien et le monomère HLA chargé en peptide, puis le tétramère muté correspondant, ont pu être obtenus suivant un protocole de renaturation précédemment décrit par Garboczi et al (3).

### Exemple 2 : Etude comparative de l'efficacité et de la spécificité de marquage immunofluorescent entre un tétramère HLA-A0201 natif et le tétramère HLA-A0201 muté chargés avec différents peptides

### a) Marquage de clones lymphocytaires

Des tétramères HLA-A0201 natifs et des tétramères correspondants mutés ont été chargés soit avec un peptide issu de la protéine BMLF1 du virus de l'EBV, soit avec un peptide issu de la protéine pp65 du CMV.

Les tétramères natifs sont obtenus selon la technique de l'exemple 1, mais sans la mutation.

Des clones spécifiques et non spécifiques ont été marqués et utilisés à des concentrations croissantes.

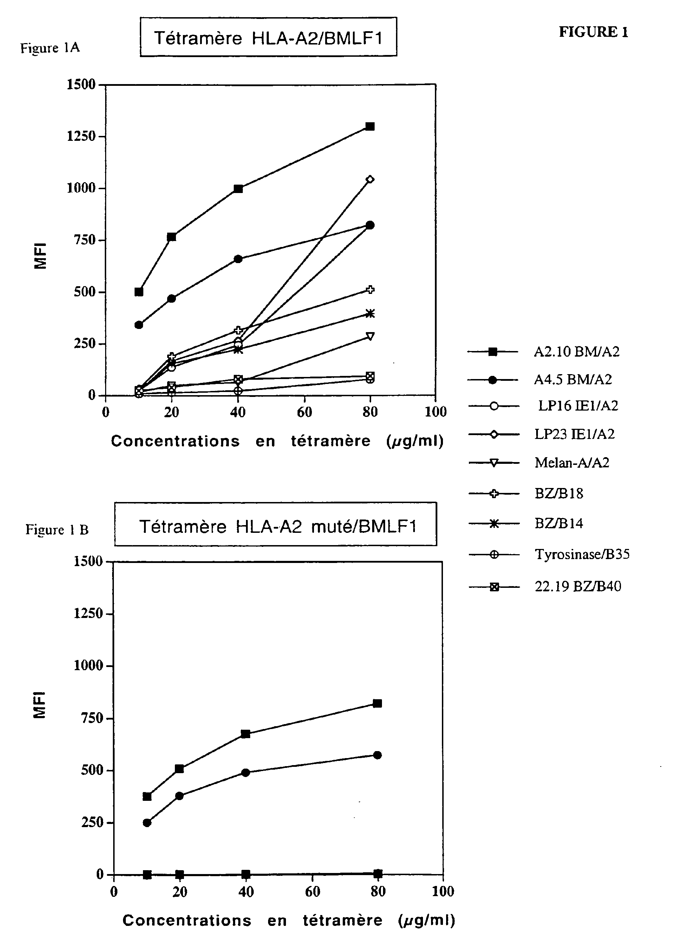

La figure 1 donne les moyennes d'intensité de fluorescence (MFI) obtenues avec les tétramères HLA-A2/BMLF1 natifs (figure 1A) et avec les tétramères correspondants mutés (figure 1B).

On constate que le tétramère HLA-A2/BM natif montre un bruit de fond de fixation sur certains clones non spécifiques qui augmente avec la dose de tétramère utilisée.

Ce bruit de fond ne semble pas exclusivement lié à la restriction HLA du clone considéré, mais dépend aussi -du peptide chargé puisque deux clones anti-IE1 restreints HLA-A0201 donnent un fort bruit de fond, alors que le clone anti-melan-A également restreint HLA-A0201 donne un bruit de fond modéré.

Avec le tétramère muté, les moyennes de fluorescence (MFI) obtenues sur les clones spécifiques (BM/A2) sont plus faibles que celles obtenues avec le tétramère natif, mais le bruit de fond sur les clones non spécifiques est quasiment nul et ceci quelle que soit la concentration de tétramère utilisée.

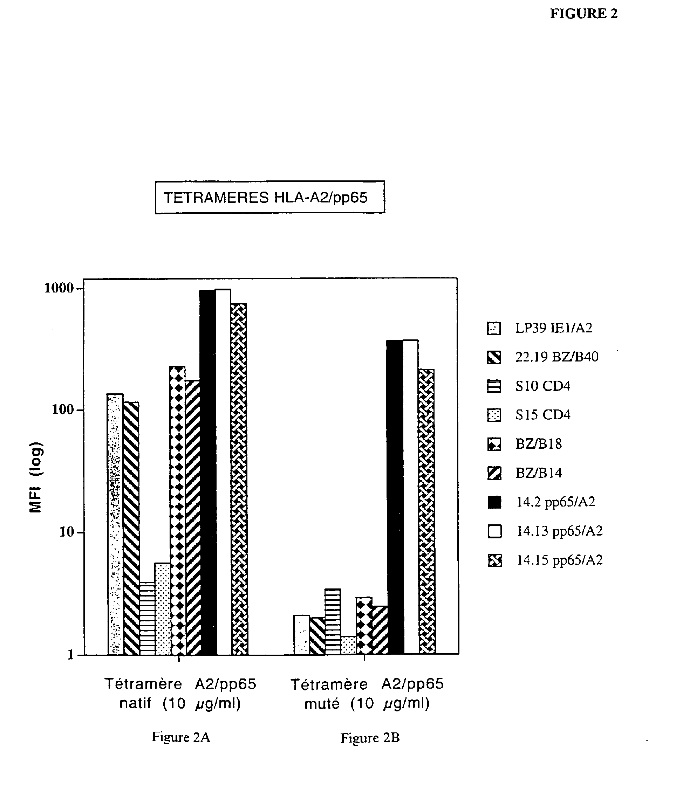

On remarquera que cette différence de bruit de fond entre le tétramère natif et le tétramère muté n'est pas particulière au tétramère A2/BM puisqu'on l'observe également avec le tétramère HLA-A2/pp65 (voir figure 2).

Dans cette figure 2, la moyenne de fluorescence est indiquée en échelle log afin de visualiser les faibles bruit de fond de marquage obtenu avec le tétramère muté. On constate que le différentiel entre le marquage spécifique et le marquage non spécifique est de l'ordre de 2 log (10²) avec le tétramère A2/pp65 muté, alors qu'il est de seulement 1 log avec le tétramère natif. Les deux exceptions notables sont les deux clones CD4 anti-EBV pour lesquels le bruit de fond est très faible avec le tétramère natif. Cette observation renforce l'hypothèse selon laquelle le bruit de fond de marquage obtenu avec le tétramère natif est dû à un certain pourcentage de fixation non spécifique du tétramère au CD8.

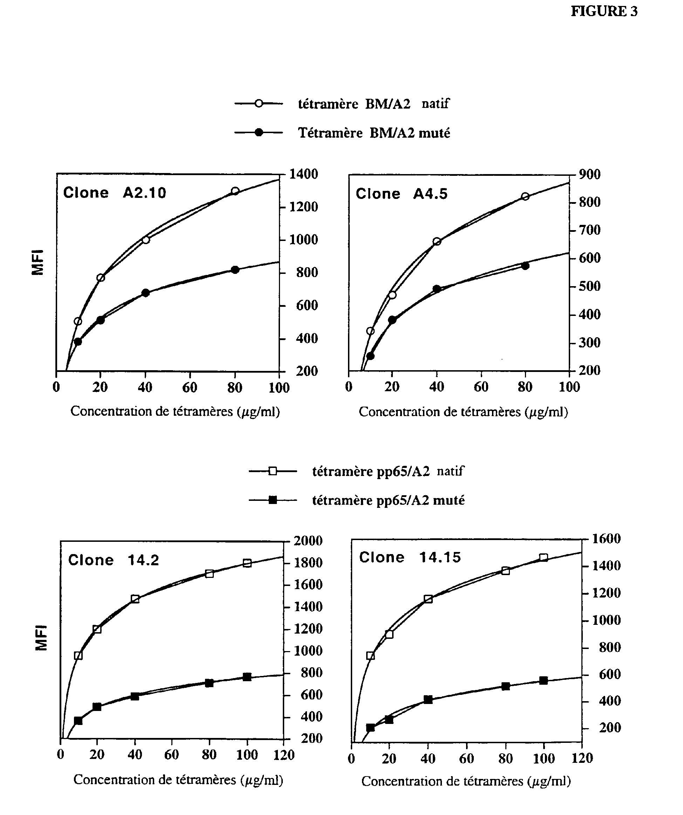

Sur la figure 3, sont représentées les différences de marquages obtenues avec les tétramères mutés et natifs chargés avec pp65 sur des clones spécifiques. La différence entre les courbes de saturation obtenues avec le tétramère muté et le tétramère natif montre qu'il existe un plus grand nombre de sites de fixation pour le tétramère natif que pour le tétramère muté sur ces clones : il est donc fortement probable que, dans le cas du tétramère natif, certaines valences interagissent avec le CD8 seul, d'autant que cette molécule est exprimée à une densité largement supérieure à celle du TCR à la surface du lymphocyte T.

D'autres expériences ont été réalisées pour tester si le marquage spécifique avec le tétramère muté était significativement affecté par le degré de CD8 dépendance de clones spécifiques. En effet, il est notoire que parmi les clones T CD8+, certains clones ont fortement besoin du CD8 pour renforcer l'interaction spécifique entre leur TCR et le complexe HLAA-peptide, tandis que d'autres clones s'en dispensent.

Le degré de CD8 dépendance a été estimé selon le test de cytotoxicité de Couedel et al (4) avec ou sans anticorps anti-CD8 et est considéré comme inversement proportionnel à l'affinité du TCR pour le complexe HLA-peptide.

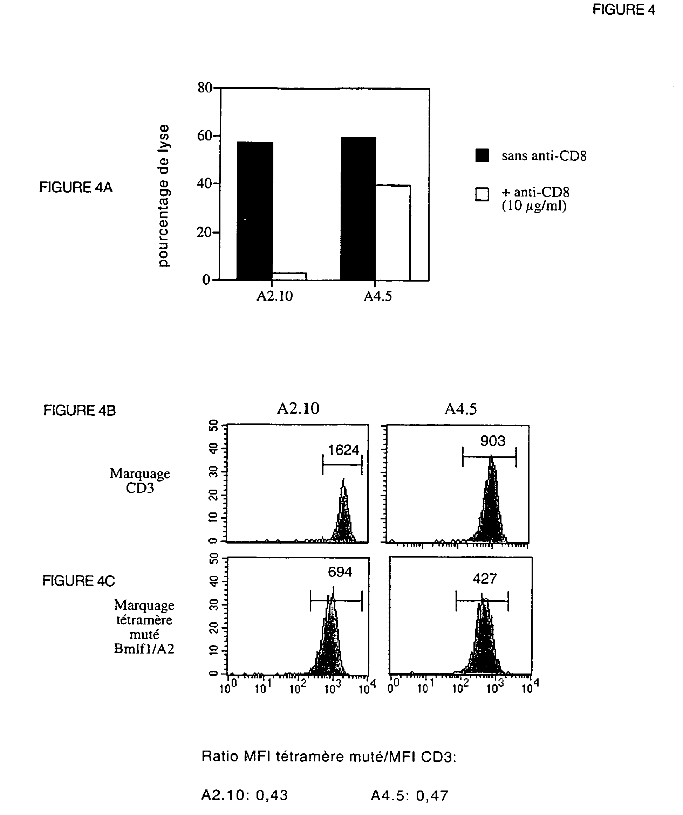

Sur la figure 4A, est représenté un test de cytotoxicité contre une lignée HLA-A0201 chargée en peptide BMLF1 (10µM) avec deux clones spécifiques. Le clone A2.10 est très CD8 dépendant puisque sa cytotoxicité est abrogée par l'anticorps anti-CD8, tandis que le clone A4.5 " est relativement peu CD8 dépendant.

Un marquage avec le tétramère muté et un marquage avec un anticorps anti-CD3 ont été réalisés pour estimer le nombre de TCR exprimés à la surface. Les résultats sont donnés sur les figures 4B (marquage CD3) et 4C (marquage du tétramère muté BMFL1/A2).

On constate que le ratio marquage tétramère/marquage CD3 est très comparable pour les deux clones, ce qui indique que le marquage avec le tétramère muté est peu affecté par le degré de CD8 dépendance (et donc par inférence, par l'affinité du TCR).

Ainsi, la diminution d'affinité pour le CD8 induite par la mutation de la molécule HLA n'affecte pas significativement le marquage spécifique du tétramère.

### b) Détection de cellules spécifiques au sein d'une population polyclonale

Pour comparer la capacité de détection du tétramère natif et muté d'un faible pourcentage de cellules spécifiques au sein d'une population polyclonale, des lymphocytes périphériques de deux patients HLA-A0201 (désignés par A et B ci-après) ont été marqués. Il s'agit de patients chez lesquels une réponse anti-peptide pp65 du CMV avait été préalablement démontrée. Ces lymphocytes avaient été préalablement amplifiés polyclonalement *in vitro* et congelés.

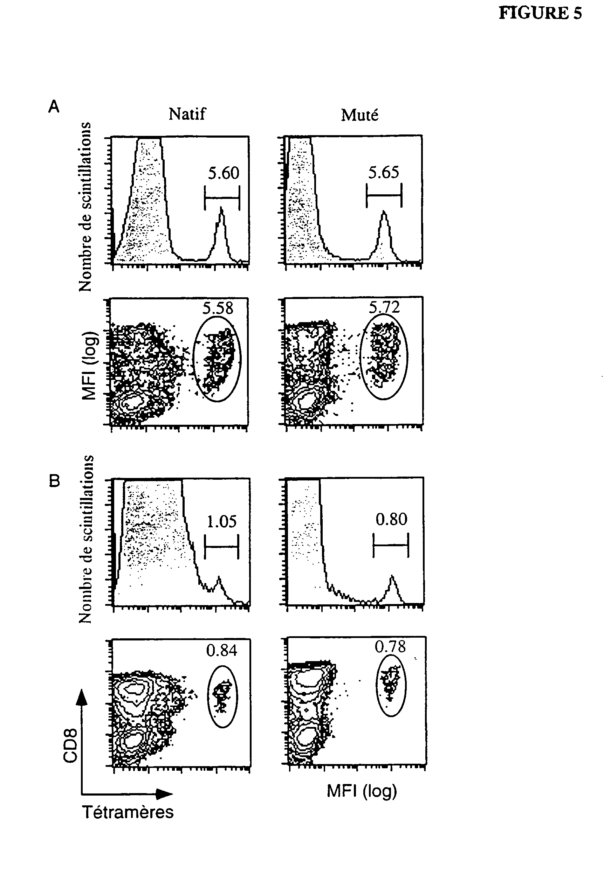

Les résultats obtenus pour les patients A et B sont reportés sur la figure 5, la figure 5A correspondant au patient A et la figure 5B au patient B. Il s'agit des résultats de simple marquage des tétramères HLA-A0201/pp65 à la phycoérythrine à 20µg/ml et des doubles marquages correspondants (tétramères marqués avec PE et des anticorps anti-CD8 marqués au FITC). Cette figure donne également les moyennes d'intensité de fluorescence (log).

On constate tout d'abord que la capacité de discrimination du tétramère natif en simple marquage est variable en fonction du pourcentage de cellules spécifiques dans la population de départ. En effet, pour le patient A, le pic de cellules positives qui représente 5,60% est aisément identifiable tandis que pour le patient B, ce pic est contaminé par du marquage non spécifique rendant difficile la détermination du pourcentage de cellules positives. En accord avec la littérature, le double marquage tétramère natif et anti-CD8 diminue le bruit de fond permettant alors d'identifier clairement la sous-population positive et d'estimer précisément son pourcentage (0,84% dans ce cas). Ceci démontre donc que l'estimation précise du pourcentage de cellules positives avec le tétramère natif implique le recours au double marquage avec l'anti-CD8.

En revanche, le simple marquage avec le tétramère muté permet chez les deux patients d'identifier sans ambiguité un pic de cellules positives. Les pourcentages de cellules positives obtenus sont quasiment identiques à ceux obtenus en double marquage avec le tétramère natif. Ceci démontre que toutes les cellules spécifiques détectables par le tétramère natif le sont également avec le tétramère muté et corrobore donc les résultats obtenus avec les clones CD8 très dépendants et peu dépendants, à savoir que la mutation n'affecte pas significativement la reconnaissance spécifique.

De plus, ces résultats montrent que le double marquage avec le tétramère muté n'apporte pas d'éléments nouveaux par rapport au simple marquage. Enfin, on peut constater que les moyennes de fluorescence obtenues avec l'anticorps anti-CD8 lors du double marquage avec le tétramère natif (216 et 193) pour les patients A et B) sont significativement plus faibles que celles obtenues en double marquage avec le tétramère muté (366 et 370 pour les patients A et B). Les moyennes de fluorescence obtenues après simple marquage de la population totale avec l'anti-CD8 étaient, respectivement, de 340 et 355 pour les patients A et B. Cela démontre la compétition de fixation entre l'anticorps anti-CD8 et le tétramère natif, compétition qui ne se produit pas avec le tétramère muté.

L'utilisation du tétramère muté permet donc de se dispenser du double marquage avec l'anti-CD8 pour estimer le pourcentage de cellules spécifiques au sein d'une population polyclonale.

### Exemple 3 : Etude comparative de l'efficacité et de la spécificité de tris immunomagnétiques réalisés avec le tétramère HLA-A0201 natif et le tétramère HLA-A0201 muté chargés avec le peptide p65.

Des monomères HLA-A0201 biotinylés et chargés en peptide pp65 ont été fixés sur des billes magnétiques couplées à la streptavidine (Dynabeads M-280 Streptavidin, DYNAL. Les populations lymphocytaires utilisées dans l'étude sont issues de liquides synoviaux ou de PBL de patients atteints de polyarthrite rhumatoide ou de PBL issus de donneurs sains séropositifs pour le CMV.

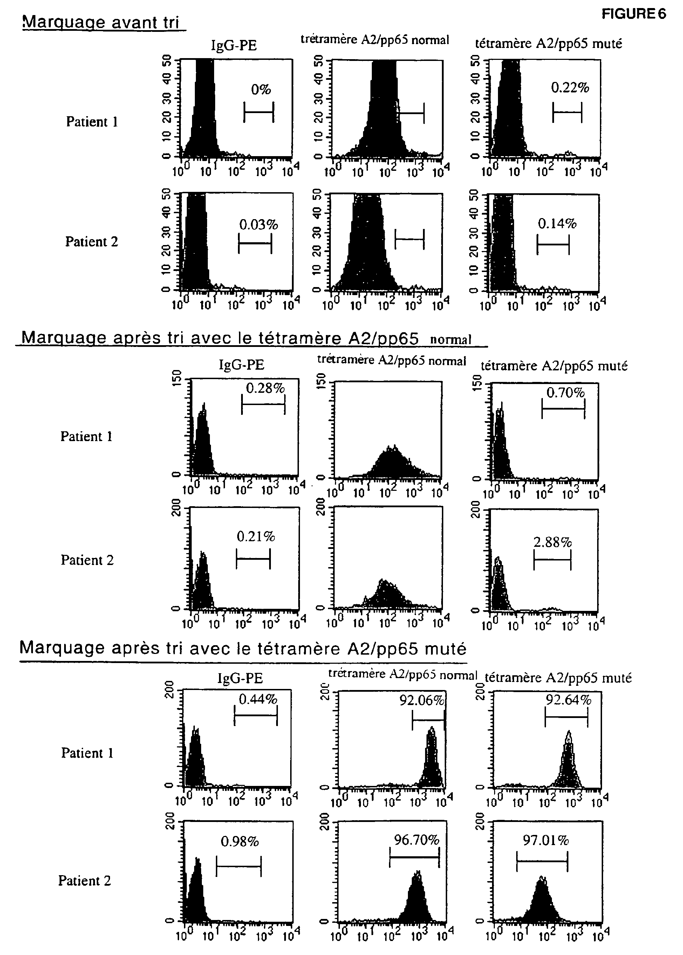

Sur la figure 6, sont indiqués les résultats d'un simple marquage avec le tétramère natif et le tétramère muté sur ces populations polyclonales. Avec le tétramère muté, il est possible de mettre en évidence un faible pourcentage de cellules positives dans les deux prélèvements (0,22 % et 0,14 %), cellules positives qui ne sont quasiment pas identifiables avec le tétramère natif.

Ces populations ont été triées avec des billes chargées soit avec le tétramère natif, soit avec le tétramère muté, puis les populations sélectionnées ont été amplifiées in vitro par une stimulation polyclonale (PHA, IL2, PBL irradiés), stimulation qui n'affecte pas la représentativité des populations amplifiées.

Un marquage a alors été réalisé sur ces populations triées et amplifiées avec les deux tétramères.

Comme représenté sur la figure 6, les cellules issues du tri avec le tétramère natif sont positivement marquées avec ce même tétramère, mais en revanche un très faible pourcentage de ces cellules sont positives avec le tétramère muté (0,7 % et 2,88 %).

Les résultats sont remarquablement différents après un tri avec le tétramère muté puisque les populations triées sont fortement positives avec les deux tétramères.

Le facteur d'enrichissement en cellules positives obtenu avec le tétramère muté est de 421 pour le prélèvement 1 (92,64 % vs 0,22 %) et de 693 pour le prélèvement 2 (97,01 % vs 0, 14 %).

Ces résultats montrent qu'en effectuant un tri avec le tétramère muté, il est possible d'obtenir ne population positive pure à plus de 90 % à partir d'un échantillon dans lequel cette population représente 0,1 à 0,2 %.

La généralité de cette observation est illustrée par le tableau ci-après, qui décrit les résultats de tris effectués avec le tétramère natif et le tétramère muté à partir de PBL et lymphocytes synoviaux de patients et donneurs sains.

**% de cellules positives de tétramères (facteur d'enrichissement)**

| Echantillons | non triés | **Premier tri avec** | | | | **Second tri avec** | | | |
|---|---|---|---|---|---|---|---|---|---|
| | | A2/pp65 | native | A2/pp65 | muté | A2/pp65 | native | A2/pp65 | muté |
| SFL 1 | 14,0 | 95,0 | (6,8) | | | | | | |
| PBL 1 | 5,6 | 89,5 | (16,0) | 97,9 | (17,5) | | | | |
| SFL 2 | 1,4 | 25,3 | (18,1) | | | 75,9 | (3,0) | | |
| PBL 2 | 0,8 | 51,1 | (63,9) | 97,5 | (121,9) | | | | |
| SFL 3 | 0,6 | 13,7 | (22,8) | | | | | | |
| PBL 3 | nd | 8,5 | (nd) | | | 12,0 | (1,4) | | |
| SFL 4 | 0,3 | 4,1 | (13,6) | | | 8,6 | (2,1) | | |
| SFL 5 | 0,2 | 0,7 | (3,5) | 92,5 | (462,5) | | | | |
| SFL 6 | 0,14 | 2,9 | (20,6) | 97,1 | (693,6) | | | | |
| PBL 4 | 0,09 | | | 82,9 | (921,1) | | | | |
| PBL 5 | 0,02 | 0,04 | (2,0) | 2,2 | (110,0) | | | 98,7 | (44,90) |

En effectuant des tris répétés avec le tétramère muté, il est possible d'obtenir des populations spécifiques pures à partir de sous-populations encore moins fréquentes (cf PBL5 dans le tableau). En revanche, ce résultat est beaucoup plus difficile à obtenir avec le tétramère natif; il semble en effet que les cellules non spécifiques isolées par un premier tri avec le tétramère natif aient une affinité suffisante pour être à nouveau sélectionnées lors des tris ultérieurs. On aboutit donc à des facteurs d'enrichissement très médiocres en réalisant un deuxième tri (cf PBL3 et SFL4).

Une autre étude a porté sur la réactivité des populations triées pour vérifier que les cellules sélectionnées pour leur capacité à fixer le tétramère A2 muté/pp65 étaient bien réactives vis-à-vis de ce complexe HLA-peptide dans un contexte physiologique.

A cet effet, on a étudié l'activation des populations lymphocytaires triées, objectivées par l'induction à la surface de la chaîne α du récepteur à l'IL2 (CD25), après mise en présence de cellules T2 (A0201+) chargées en peptide pp65.

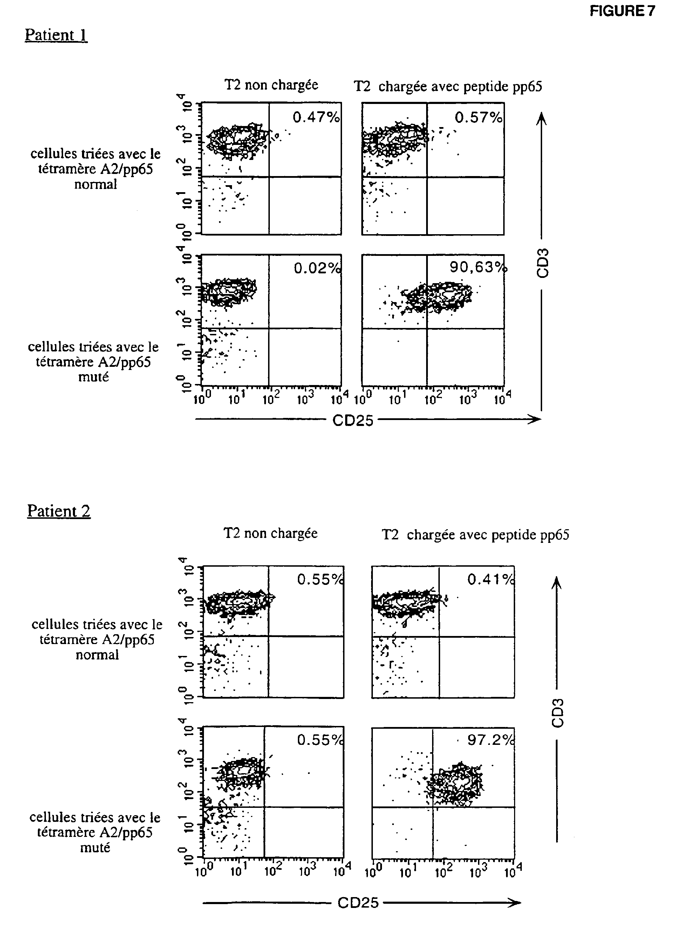

Comme le montre la figure 7, les populations triées à l'aide du tétramère natif n'expriment pas le CD25 après contact avec les cellules T2 chargées en peptides.

En revanche, la majorité des cellules triées avec le tétramère muté s'activent en présence de T2 chargées avec le peptide pp65 et le pourcentage de cellules positives en CD25 correspond bien avec le pourcentage de cellules qui étaient marquées par le tétramère muté (90,63 % de cellules activées vs 92,64 % de cellules marquées et 97,20 % vs 97,01 % pour les patients 1 et 2 respectivement).

Ces résultats démontrent que les cellules marquées et triées avec le tétramère muté sont exclusivement des cellules réactives, tandis que les cellules triées avec le tétramère natif sont non réactives.

Le fait qu'on ne retrouve aucune cellule réactive après tri avec le tétramère natif alors qu'on parvenait à distinguer quelques cellules positives dans cette population est vraisemblablement dû au pourcentage trop faible de ces cellules pour être détectables dans un test fonctionnel.

L'ensemble de ces résultats démontre la supériorité manifeste du tétramère muté par rapport au tétramère natif pour sélectionner par tri immunomagnétique des populations faiblement représentées dans la population de départ.

Lorsque les populations spécifiques sont plus nombreuses dans le prélèvement de départ, il devient alors possible d'isoler des cellules réactives avec le tétramère natif, mais les degrés de pureté des populations obtenues sont très inférieurs à ceux obtenus en parallèle avec le tétramère muté (tableau).

### REFERENCES BIBLIOGRAPHIQUES

1) Altman J.D. et al, 1996, Science 274:94-6 et brevet US 5 635 363
2) Salter, R.D. et al, 1990, Nature 345:41-46
(3) Garboczi D.N. et al, 1992, Proc Natl Acad Sci USA 89:3429-33
4) Couedel, C., M. et al, 1999, J. Immunol 162-6351-8.
5) Luxembourg A.T. et al, Nature Biotechnology, vol 16, mars 1998, 281-285.

## Revendications

1. Complexe tétramérique de monomères de CMH de classe I ayant au moins une substitution d'un acide aminé dans le domaine α3 dans la zone d'interaction d'une chaîne lourde de monomère de CMH de classe I avec un co-récepteur CD8 des lymphocytes T, l'affinité de ce complexe tétramérique pour les co-récepteurs CD8 des lymphocytes T étant réduite par rapport à l'affinité d'un complexe tétramérique natif non substitué correspondant.

2. Complexe tétramérique selon la revendication 1, **caractérisé en ce que** la substitution est dans un domaine α3 de la chaîne lourde.

3. Complexe tétramérique selon la revendication 1 ou 2, **caractérisé en ce que** les monomères de CMH sont sous forme d'un complexe avec un peptide se liant au CMH.

4. Utilisation d'un complexe tétramérique selon l'une quelconque des revendications 1 à 3, pour détecter et/ou isoler des populations de lymphocytes T CD8⁺ peptide-spécifiques.

5. Utilisation d'un complexe tétramérique selon la revendication 4, dans une méthode de tri cellulaire.

6. Utilisation d'un complexe tétramérique selon la revendication 5, **caractérisée en ce que** la méthode de tri cellulaire est une méthode de tri immunomagnétique.

7. Méthode pour détecter des populations de lymphocytes T CD8⁺ peptide-spécifiques dans une population polyclonale, **caractérisée en ce qu'**elle comprend :
- la mise en contact de la population polyclonale avec les complexes tétramériques selon l'une quelconque des revendications 1 à 3, dans des conditions permettant l'interaction entre les complexes monomères de CMH de classe I recombinant /peptide et les récepteurs de lymphocytes T ayant une affinité pour lesdits complexes ; et
- la détection des lymphocytes T ayant une affinité pour lesdits complexes, pour détecter la population CD8⁺ peptide-spécifique.

8. Méthode selon la revendication 7, **caractérisée en ce que** les complexes de CMH de classe I tétramères/peptide comprennent une entité fluorescente et que la détection comprend la détection de la fluorescence desdits complexes.

9. Méthode pour isoler des populations de lymphocytes T CD8⁺ peptide-spécifique à partir d'une population polyclonale, **caractérisée en ce qu'**elle comprend :
- la mise en contact de la population polyclonale avec des billes magnétiques auquelles sont liées les complexes tétramériques selon l'une quelconque des revendications 1 à 3, dans des conditions permettant l'interaction entre les complexes monomère de CMH de classe I recombinant /peptide et les récepteurs de lymphocytes T ayant une affinité pour lesdits complexes ; et
- la récupération des lymphocytes T liés pour isoler la population CD8⁺ peptide-spécifique

## Claims

1. A tetrameric complex of MHC class I monomers having at least one aminoacid substitution in the α3 domain in the zone of interaction of a monomer heavy chain of MHC class I with a CD8 co-receptor of T lymphocytes, the affinity of the said tetrameric complex for the CD8 co-receptors of T lymphocytes being reduced as compared to affinity of a corresponding unsubstitued native tetrameric complex.

2. The tetrameric complex according to claim 1, **characterized in that** the substitution is in an α3 domain of the heavy chain.

3. The tetrameric complex according to claim 1 or 2, **characterized in that** the MHC monomers are in the form of a complex with a MHC-binding peptide.

4. Use of the tetrameric complex of anyone of claims 1 to 3, for detecting and/or isolating peptide-specific CD8⁺ T-lymphocyte populations.

5. Use of the tetrameric complex according to claim 4, in a cell-sorting method.

6. Use of the tetrameric complex according to claim 5, wherein the cell-sorting method is an immunomagnetic sorting method.

7. A method for detecting peptide-specific CD8⁺ T-lymphocyte populations within a polyclonal population comprising:
- contacting the polyclonal population with tetrameric complexes of anyone of claims 1 to 3, under conditions that allow interaction between the recombinant class I MHC monomer/peptide complexes and T-lymphocyte receptors that have an affinity for said complexes; and
- detecting T-lymphocytes that have an affinity for said complexes to detect the peptide-specific CD8⁺ population.

8. The method of claim 7, wherein the class I MHC tetramer/peptide complexes comprise a fluorescent entity and the detecting comprises detecting fluorescence of said complexes.

9. A method for isolating peptide-specific CD8⁺ T-lymphocyte populations from a polyclonal population comprising:
- contacting the polyclonal population with a magnetic bead to which are bound tetrameric complexes of anyone of claims 1 to 3, under conditions that allow interaction between the recombinant class I MHC monomer/peptide complexes and T-lymphocyte receptors that have an affinity for said complexes; and
- recovering bound T-lymphocytes to isolate the peptide-specific CD8⁺ population.

## Patentansprüche

1. Tetramerer Komplex von MHC-Klasse-I-Monomeren, die wenigstens eine Substitution einer Aminosäure in der α3-Domäne in der Zone der Wechselwirkung einer schweren Kette eines MHC-Klasse-I-Monomers mit einem CD8-Corezeptor von T-Lymphocyten aufweisen, wobei die Affinität dieses tetrameren Komplexes zu den CD8-Corezeptoren von T-Lymphocyten in Bezug auf die Affinität eines entsprechenden unsubstituierten nativen tetrameren Komplexes reduziert ist.

2. Tetramerer Komplex gemäß Anspruch 1, **dadurch gekennzeichnet, dass** sich die Substitution in einer α3-Domäne der schweren Kette befindet.

3. Tetramerer Komplex gemäß Anspruch 1 oder 2, **dadurch gekennzeichnet, dass** die MHC-Monomere in Form eines Komplexes mit einem MHCbindenden Peptid vorliegen.

4. Verwendung eines tetrameren Komplexes gemäß einem der Ansprüche 1 bis 3 zum Nachweisen und/oder Isolieren der peptidspezifischen Populationen von CD8-positiven T-Lymphocyten.

5. Verwendung eines tetrameren Komplexes gemäß Anspruch 4 in einem Zellsortierungsverfahren.

6. Verwendung eines tetrameren Komplexes gemäß Anspruch 5, **dadurch gekennzeichnet, dass** das Zellsortierungsverfahren ein immunmagnetisches Sortierungsverfahren ist.

7. Verfahren zum Nachweisen der peptidspezifischen Populationen von CD8-positiven T-Lymphocyten in einer polyklonalen Population, **dadurch gekennzeichnet, dass** es Folgendes umfasst:
- das In-Kontakt-Bringen der polyklonalen Population mit den tetrameren Komplexen gemäß einem der Ansprüche 1 bis 3 unter Bedingungen, die eine Wechselwirkung zwischen den Komplexen aus rekombinanten MHC-Klasse-I-Monomeren und Peptid und den Rezeptoren von T-Lymphocyten mit einer Affinität zu diesen Komplexen erlauben; und
- den Nachweis der T-Lymphocyten mit einer Affinität zu diesen Komplexen, so dass die peptidspezifische CD8-positive Population nachgewiesen wird.

8. Verfahren gemäß Anspruch 7, **dadurch gekennzeichnet, dass** die Komplexe aus tetrameren Klasse-I-MHC und Peptid eine fluoreszierende Struktureinheit umfassen und dass der Nachweis den Nachweis der Fluoreszenz der Komplexe umfasst.

9. Verfahren zum Isolieren der peptidspezifischen Populationen von CD8-positiven T-Lymphocyten ausgehend von einer polyklonalen Population, **dadurch gekennzeichnet, dass** es Folgendes umfasst:
- das In-Kontakt-Bringen der polyklonalen Population mit Magnetkügelchen, an die die tetrameren Komplexe gemäß einem der Ansprüche 1 bis 3 gebunden sind, unter Bedingungen, die eine Wechselwirkung zwischen den Komplexen aus rekombinantem MHC-Klasse-I-Monomer und Peptid und den Rezeptoren von T-Lymphocyten mit einer Affinität zu diesen Komplexen erlauben; und
- die Gewinnung der gebundenen T-Lymphocyten, so dass die peptidspezifische CD8-positive Population isoliert wird.
